# EUROPEAN PATENT APPLICATION

(11) **EP 4 541 383 A2**
(43) Date of publication of application: **23.04.2025**
(21) Application number: 24203095.5
(22) Date of filing: 27.09.2024
(51) Int. Cl.: A61L 2/26, A61B 50/30, A61L 2/28, B65B 55/02, B65B 55/16, A61L 2/10

(54) **PACKAGING FOR A STERILIZABLE OBJECT**

(30) Priority: 27.09.2023 BE 202305800
(71) Applicant: Eledricity BV, 9820 Merelbeke (BE)
(72) Inventor: VERSTRAETEN, Kilian-Duncan, 9160 Lokeren (BE)
(74) Representative: Brantsandpatents bv

(57) **Abstract**

The current invention relates to a packaging for a sterilizable object, comprising: a bag, which bag comprises at least two opposing walls, wherein each wall has a thickness of at least 10 µm and at most 60 µm and is made of an ultraviolet-C (UVC) transmissive thermoplastic polymer, the at least two walls defining a first compartment for receiving the sterilizable object; and a UVC indicator, which UVC indicator comprises a photochromic ink suitable for changing color after exposure to UVC radiation. The invention also relates to a use of the packaging.

## Description

### TECHNICAL FIELD

The invention relates to a packaging for a disinfectable or sterilizable object.

In another aspect, the invention also relates to a use for transporting disinfected or sterilized objects.

### PRIOR ART

In healthcare, it is crucial to prevent the spread of pathogens by ensuring that frequently touched and reused objects, such as medical equipment and patient care items, are thoroughly disinfected or sterilized after each use. This process is essential to prevent infections and maintain overall hygiene in the care environment.

Medical and surgical instruments are often sterilized or disinfected using steam, one of the most effective and widespread sterilization methods. The steam sterilization process involves placing the instruments in specially designed containers that contain steam. The steam is then heated to a certain temperature and pressure, which kills microorganisms and other potentially harmful elements on the instruments. After the sterilization process or disinfection process, the sterilized or disinfected instruments must be handled carefully to maintain the sterile environment. This involves a sterile individual packing the instruments in an aseptic environment. This process is vital to ensure that the instruments maintain their sterile status until the time of use. Failure to comply with strict sterilization and packaging protocols will increase the risk of infections in patients, as recontamination will occur if the instrument is not handled correctly after the sterilization process. Despite all precautions, there remains a constant risk of recontamination.

For this purpose, ultraviolet (UV) light can also be used to disinfect objects by damaging the DNA and RNA of microorganisms. More specifically, UVC disinfectors, also called UVC light disinfection systems, use ultraviolet-C radiation (UVC radiation) with a wavelength between 200 and 280 nanometers to kill or inactivate microorganisms such as bacteria, viruses, fungi and other pathogens. This type of radiation is powerful enough to damage the DNA and RNA of these microorganisms, causing them to lose their ability to reproduce and maintain their harmful effects.

However, the use of UVC radiation does pose some problems. UVC radiation is harmful to the skin and eyes. Prolonged exposure can lead to skin burns, eye irritation, and in more severe cases, damage to the retina. UVC radiation has a limited range and requires direct exposure to the surfaces or air to be disinfected. Shaded areas or inaccessible areas may not receive sufficient UVC radiation for effective disinfection. During this disinfection process, areas where UVC rays are used must be carefully shielded to prevent people or animals from being unintentionally exposed to the radiation.

In addition to these challenges with UVC radiation, there are also specific problems related to transporting and maintaining objects as disinfected or sterilized. The transport of disinfected or sterilized objects will be a challenge, especially in situations where disinfection takes place outside a controlled environment. During transportation, objects can be re-exposed to microorganisms or contaminants, reducing the original disinfection level. Moreover, maintaining the level of disinfection or sterilization over longer periods is difficult, especially when objects need to be stored or transported before use. Factors such as environmental conditions, exposure to air, humidity, and contact with non-disinfected surfaces can reduce effectiveness. The risk of recontamination during transport is significant because objects may come into contact with surfaces or environments that have not been disinfected, which can lead to a loss of the achieved hygiene standard. In addition, disinfected or sterilized objects can be mixed up during transport, which entails life-threatening risks.

The present invention aims to at least find a solution to some of the above-mentioned problems or disadvantages. The aim of the invention is to provide a method which eliminates those disadvantages.

### SUMMARY OF THE INVENTION

In a first aspect, the present invention relates to a packaging according to claim 1. Preferred embodiments of the device are shown in claims 2 to 13.

The packaging is extremely suitable for sterilizing or disinfecting objects and then transporting them safely without the risk of recontamination. This will help prevent the spread of infections. First of all, the use of UVC transmissive thermoplastic polymer allows efficient disinfection and sterilization due to its permeability to UVC radiation. This feature is crucial to ensure that objects are thoroughly rid of germs and bacteria without re-exposing them to potential contamination after the process since the air present in the sealed packaging is also disinfected or sterilized. In addition, the ideal balance between flexibility and durability is achieved by precisely specifying the wall thickness of at least 10 µm and at most 60 µm. This allows the packaging to retain its strength while the UVC radiation effectively penetrates and does its job. The presence of an indicator, using photochromic ink that changes color after exposure to UVC radiation, provides a clear visual representation of the disinfection process. This contributes to the confidence in the effectiveness of the disinfection procedure, allowing users to quickly and easily verify if the desired level of disinfection has been achieved. This cleverly designed packaging combines effective disinfection with a reliable indication, making it an optimal packaging for the safe and efficient disinfection and/or sterilization of objects. The emphasis here is on both health and hygiene aspects.

In a second aspect, the present invention relates to a use according to claim 14.

The use of this packaging is particularly advantageous because it enables and maintains the disinfection and sterilization of objects, especially for medical instruments and food. This contributes to patient safety and food safety, reduces spoilage and waste, and improves the efficiency of distribution.

### DESCRIPTION OF THE FIGURES

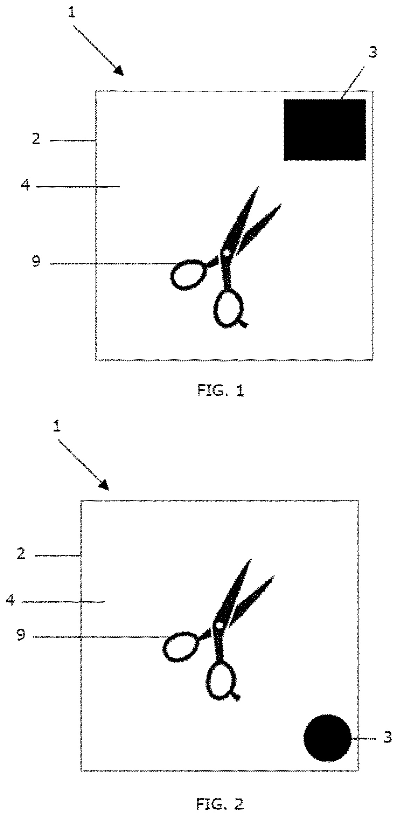

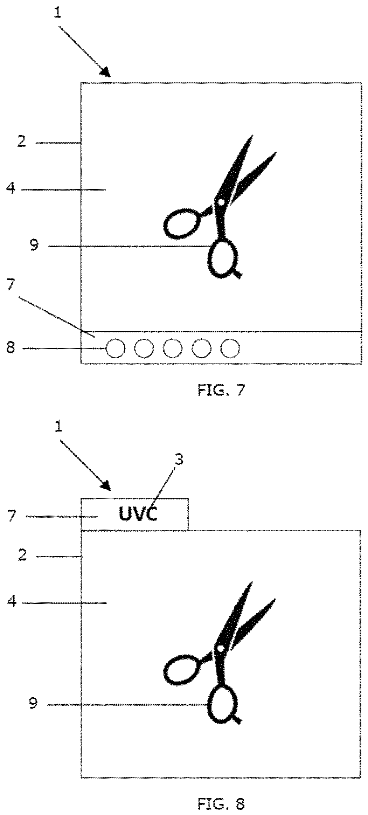

**Figures 1 to 8** show a schematic front view of different embodiments of the present invention.

### DETAILED DESCRIPTION

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, "a" and "the" refer to both the singular and the plural, unless the context presupposes otherwise. For example, "a segment" means one or more segments.

When the term "around" or "about" is used in this document with a measurable quantity, a parameter, a duration or moment, and the like, then variations are meant of approx. 20% or less, preferably approx. 10% or less, more preferably approx. 5% or less, even more preferably approx. 1% or less, and even more preferably approx. 0.1% or less than and of the quoted value, insofar as such variations are applicable in the described invention. However, it must be understood that the value of a quantity used where the term "about" or "around" is used, is itself specifically disclosed.

The terms "comprise," "comprising," "consist of," "consisting of," "provided with," "include," "including," "contain," "containing," are synonyms and are inclusive or open terms that indicate the presence of what follows, and which do not exclude or prevent the presence of other components, characteristics, elements, members, steps, as known from or disclosed in the prior art.

Quoting numeric intervals by the endpoints includes all integers, fractions, and/or real numbers between the endpoints, including those endpoints.

The term 'UVC radiation' refers to the part of the electromagnetic spectrum that consists of ultraviolet radiation with the shortest wavelengths and the highest energy. UVC radiation varies in wavelength from approximately 200 to 280 nanometers (nm). Due to its high energy, UVC radiation has disinfectant properties and is often used for germ and virus destruction, disinfection processes, and laboratory applications.

The term "appendage" refers to an additional part or component that is included in the design of the bag. In this case, the "appendage" is the specific part of the bag on which the UVC indicator is provided. Such an appendage can either be a separate section attached to the bag (e.g., a label) or can be an already present part of the bag that is functionally separate (e.g., a seam).

The terms "disinfected object" and "sterilized object" refer to objects or materials that have been treated to reduce or completely eliminate microorganisms. A "disinfected object" is an object whose surfaces have been treated with disinfectants, such as UVC radiation, to reduce harmful microorganisms to a safe level. An "sterilized object" is an object in which all microorganisms, including bacteria, viruses and spores, have been completely killed or inactivated, making it completely germ-free. "Disinfecting" means the reduction of the number of microorganisms on surfaces or objects to a level at which they do not pose a health hazard. This process kills or inactivates most microorganisms, but possibly not all bacterial spores. "Sterilization" goes beyond disinfection and means the complete killing or inactivation of all microorganisms, including spores. Sterilization ensures an absolutely germ-free result and is often applied in medical and laboratory environments. "Disinfectable" means that a surface, object or material is suitable for being disinfected, which is essential to prevent the spread of pathogens. 'Sterilizable' indicates that something can be sterilized to ensure that it is completely free of microorganisms.

The term "transparent" is synonymous with "substantially light-transmitting." If something is transparent, it means that it allows light or radiation to pass through without substantial scattering, which often results in the object or material being colorless or appearing so, although not necessarily. In this case, the transparency refers to the transmission of UVC radiation.

The term "compartment" refers to a separate and often distinct or demarcated space, section or portion of a specific object, in this context the bag of the packaging.

A first aspect the invention relates to a method for packaging for a sterilizable object.

According to a preferred embodiment, the packaging comprises a bag. The bag comprises at least two opposing walls. The at least two walls defining a first compartment for receiving the sterilizable or disinfectable object. By placing the sterilizable or disinfectable object in a compartment, the interior space of the packaging is organized, and it protects the object from possible damage due to friction or contact with other objects. This is important among other things for sensitive or fragile items. The main advantage of separating into a specific compartment is that it helps maintain hygiene and reduces the risk of recontamination during transport by keeping the object in a sterile compartment. A crucial aspect is therefore that the object in the packaging is disinfected or sterilized and then no longer exposed to potentially contaminated air. A specific advantage of compartmentalizing the packaging is avoiding that multiple objects end up on or under each other. UVC radiation can only ensure its effectiveness if there is no shadow formation. The radiation must be able to reach each surface unhindered. A known problem with UVC disinfection is that multiple objects lie too close or on top of each other, resulting in shadow formation, and thus no disinfection occurs.

According to a preferred embodiment, the first compartment is provided with at least one opening. This opening makes it possible to easily place the object in the compartment. By providing one opening, the sterility of the object will be easier to maintain. Preferably, the opening can also be sealed, which provides the possibility to maintain a sterile environment. The closure of the opening ensures that the object is protected from external contamination, thereby maintaining a sterile environment. This is particularly crucial in medical, pharmaceutical, or laboratory applications, where maintaining sterile conditions is essential. Closing the opening contributes to the safety of the object and possibly the user. It prevents the object from unintentionally falling out of the compartment or being exposed to contaminated or infected objects and/or to contaminated ambient air. The opening of the bag can be sealed in different ways. This includes, among other things, the use of an elastic band, a paperclip or binder clip, special plastic clips or bag ties, sealing the opening by means of heat, folding the opening multiple times, closing it with a hook-and-loop fastener, or providing it with a ziplock closure.

According to a preferred embodiment, each wall consists of a UVC transmissive thermoplastic polymer, and each wall has a thickness of at least 10 µm and at most 60 µm, preferably at least 15 µm and at most 58 µm, more preferably at least 20 µm and at most 56 µm, even more preferably at least 25 µm and at most 54 µm, even more preferably at least 30 µm and at most 52 µm, even more preferably at least 35 µm and at most 50 µm, even more preferably at least 36 µm and at most 48 µm, even more preferably at least 37 µm and at most 46 µm, even more preferably at least 38 µm and at most 44 µm, even more preferably at least 39 µm and at most 42 µm, and particularly about 40 µm. This optimal wall thickness, made of UVC transmissive thermoplastic polymer, plays a crucial role in allowing UVC radiation to pass through the bag wall for effective disinfection, while also maintaining structural integrity. The optimal thickness is determined by finding a balance between sufficient strength of the material and maximum transmission of UVC radiation.

The applicants have discovered that this thickness is optimal to ensure that objects are properly disinfected and can be transported free of contamination. This is of great importance for preventing the spread of infections. With the proposed maximum thickness of the wall of the bag, a high transmission (low absorption) of UVC radiation is ensured, which is essential for effective disinfection. At the same time, the wall has a sufficiently high minimum thickness to ensure structural integrity and prevent the material from becoming weak or vulnerable. As a result, the walls will retain the necessary shape and be durable in use, even when exposed to UVC radiation and any external forces. Particularly in medical applications, it is often the case that these have sharp or pointed ends, even though they are equipped with a cap or another closure (scissors, syringes, scalpels, etc.). In this way, there is a risk that the objects will cut open/break the bag itself, thereby causing a risk of contamination during further storage, transport, etc.

If the walls of the bag are thinner than this optimal thickness, the strength and durability of the bag will decrease. A wall that is too thin will be more easily damaged or torn when exposed to stress, impact, or repeated use. This increases the risk of recontamination of the disinfected object.

On the other hand, if the walls are thicker than the optimized thickness, this will reduce the passage of UVC radiation. A thicker wall will absorb more UVC radiation instead of allowing it to pass through, making the disinfection process less effective.

According to a further or alternative embodiment, each wall of the bag is made from a polypropylene (PP), preferably from a biaxially oriented polypropylene (BOPP), more preferably from a fluorinated ethylene propylene (FEP). PP is naturally permeable to UVC rays. It is a versatile polymer that offers good optical transparency and has relatively low absorption of UVC radiation. This allows UVC radiation to pass through effectively, making it suitable for disinfection applications. BOPP is a form of polypropylene in which the polymer is stretched in two directions during the production process. This improves the clarity and optical properties, making it an excellent material for transmitting UVC radiation without significant absorption. FEP is a polymer that contains fluorine, giving it excellent transparency and permeability for UVC radiation. It also has good thermal stability and chemical resistance, making it suitable for applications where exposure to high temperatures and chemical environments is possible.

According to an alternative embodiment, each wall of the bag is made of polyethylene, polycarbonate, polyvinyl chloride, polyethylene terephthalate, polyurethane, polytetrafluoroethylene, or ethylene tetrafluoroethylene. Polyethylene is a commonly used plastic and may be suitable for the manufacture of UV-permeable bags. It is available in various densities and can effectively transmit UVC rays. Polycarbonate is a strong and durable material that can transmit UVC rays. It is often used in applications where both strength and optical clarity are important. polyvinyl chloride is known for its versatility and can be adapted to allow UVC rays to pass through while remaining sturdy. Polyethylene terephthalate is lightweight and has good optical properties. Polyurethane can be used for making flexible and UVC-permeable bags. It can have different thicknesses, depending on the application. Polytetrafluoroethylene has good chemical resistance and transparency to UVC rays. Ethylene tetrafluoroethylene is a high performance polymer that offers excellent optical clarity and effectively transmits UVC rays. It is often used in applications where high performance and durability are required.

According to a preferred embodiment, the packaging comprises a UVC indicator, which UVC indicator comprises a photochromic ink suitable for changing color after exposure to UVC radiation. The photochromic ink is designed to change color when exposed to UVC radiation, providing a visual indication of the sterilization. This change in color indicates a successful sterilization, allowing users to immediately and visually confirm whether the object in the packaging has been thoroughly sterilized.

According to a further embodiment, the UVC indicator is a photochromic ink, which ink comprises a mixture selected from the list of: photoactive compounds or the active photochromic element, binders, solvents, stabilizers, additives, dyes, or a combination thereof. Photoactive compounds change color when exposed to UVC radiation. The binders are polymers that bind the ink together and adhere it to the surface to which it is applied. They contribute to the durability and stability of the ink. The solvents may be organic solvents such as ethyl acetate, toluene, or acetone. Solvents are used to dissolve the ink components and form a homogeneous solution. Stabilizers and additives such as antioxidants and UV stabilizers can be added to improve the stability, durability, and color intensity of the ink. Finally, dyes can be added to obtain the desired color when the ink reacts to the UVC radiation.

According to an alternative or further embodiment, the UVC indicator comprises a photo-active compound selected from the list of: spirobenzopyrans, fulgide derivatives, dihydroindolizine derivatives, naphthopyrans or spiropyrans. These substances are often used as the active photochromic element in ink formulations. The choice of the specific photochromic molecule depends on factors such as the desired color change, sensitivity to UV radiation, stability, and application of the indicator. Spirobenzopyrans are common photochromic substances that change color when exposed to UV radiation. They can serve as the basis for photochromic ink. Fulgide is another type of photochromic compound used for its sensitivity to UV radiation and ability to change color. Dihydroindolizine derivative compounds also have photochromic properties and can be used to produce ink that reacts to UV radiation. Naphthopyrans are photochromic substances that light up and change color when exposed to UV radiation. Spiropyrans can change into merocyanine when exposed to UV radiation, making them suitable for photochromic applications. These substances are often used as the active photochromic element in ink formulations. The choice of the specific photochromic molecule depends on factors such as the desired color change, sensitivity to UV radiation, stability, and application of the indicator.

More preferably, the photoactive compounds are spirochromic compounds or spiro compounds, such as spirooxazine or spiro[indoline-2.2'-phthalide]. Spirooxazine is a commonly used photochromic compound that changes from a colorless or lightly colored form to a colored form under the influence of UV radiation. Spiro[indoline-2.2'-phthalide], also known as SPI, is another type of spiro compound used in photochromic applications. It undergoes a color change when exposed to UV radiation.

According to a further or alternative embodiment, the UVC indicator comprises a solvent-based photochromic ink. Preferably, the solvent-based photochromic ink comprises a mixture of a solvent and a UVC-sensitive pigment. The solvent or dissolving agent serves as a simple solution for proper printing or application in other ways, while the pigment provides the coloration. Preferably, this is in a ratio of at least 45 wt% and at most 95 wt% base ink and at least 5 wt% and at most 55 wt% pigment, more preferably at least 50 wt% and at most 90 wt% solvent and at least 10 wt% and at most 50 wt% pigment, even more preferably at least 55 wt% and at most 88 wt% solvent and at least 12 wt% and at most 45 wt% pigment, even more preferably at least 60 wt% and at most 86 wt% solvent and at least 14 wt% and at most 40 wt% pigment, even more preferably at least 65 wt% and at most 84 wt% solvent and at least 16 wt% and at most 35 wt% pigment, even more preferably at least 70 wt% and at most 82 wt% solvent and at least 18 wt% and at most 30 wt% pigment, still even more preferably at least 75 wt% and at most 80 wt% solvent and at least 20 wt% and at most 25 wt% pigment. This mixture is particularly suitable for use on both polyethylene and polypropylene surfaces.

Solvents in ink perform multiple important functions that contribute to the properties and performance of the ink. First of all, they have the ability to dissolve various components of the ink, such as resins, pigments, dyes, and other additives. This solution ensures a homogeneous and consistent ink mixture. Moreover, solvents serve to dilute the ink to the desired viscosity, making it easier to apply the ink and allowing it to flow through printing equipment. Another essential aspect is the dispersion of pigment particles in the ink. Moreover, some solvents can evaporate quickly, causing the ink to dry faster and making the printing process more efficient. In addition, solvents can be adjusted to influence the drying curve of the ink to suit the printing process and the substrate being printed on. Preferably, the solvent is selected from the list of: cyclohexanone, chloromethane (chloroform), toluene, methyl isobutyl ketone (MIBK), dimethylformamide (DMF), dimethyl acetate (DMA), acetone, ethanol, isopropanol, butanone, ethyl acetate, xylene, petroleum ether, propylene glycol, diacetone, methanol, cellosolve (ethylene glycol monomethyl ether) or a combination thereof.

The high content of pigment is intended to promote a strong reaction of the ink to UVC radiation, resulting in the desired color change. The mentioned concentrations also contribute to the chemical stability of the ink, ensuring it remains effective for a long time. Simultaneously, the added pigment content plays a crucial role in maintaining the color intensity and coverage of the ink when it is not exposed to UVC radiation.

An excessive amount of solvent can lead to reduced color intensity, longer drying time, and bleeding on the surface. It can also disrupt the consistency of the ink. On the other hand, too little solvent results in a viscosity that is too high, causing problems in flowing through printing equipment and uneven coverage. This can also cause rapid drying and reduced adhesion to the substrate. The right balance between a solvent and the pigment is essential to obtain an ink that has good fluidity, good color coverage and is suitable for the intended printing process and substrate. The same principle applies to the pigment content: a higher amount of pigment can increase the color intensity, but also raise the viscosity, while less pigment results in a weaker color, but at the same time can reduce the viscosity. Finding the right balance is essential to determine the optimal proportions that correspond to the desired properties of the ink and the intended applications. By carefully maintaining the balance between the solvent and the pigment, a practical and effective mixture is created that excels in various applications and ensures the desired visual and photochromic properties on many surfaces, particularly polyethylene and polypropylene.

According to a further or alternative embodiment, the UVC indicator comprises a solvent-based photochromic ink, which solvent-based photochromic ink comprises a phosphate. Preferably, the phosphate is chosen from the list of: triphenyl phosphate, tripolyphosphate, disodium phosphate, sodium aluminum phosphate, ammonium phosphate, phosphoric acid, spirooxazine phosphate, dithienylethenyl phosphate, peri-conjugated phosphorous ester, naphthopyran phosphate, chalcone phosphate. These phosphates form a part of solvent-based photochromic inks, where their chemical properties and photochromic reactions are carefully balanced to achieve the desired color changes and optical characteristics. Phosphates are added to inks because of their versatile technical properties. First, phosphates can serve as viscosity regulators, giving the ink the desired flowability for easy processing. Moreover, phosphates serve as pH stabilizers, essential to ensure the chemical stability of the ink. Some phosphates have reactive properties and function as catalysts or reagents for chemical reactions in the ink. They stabilize pigments and dyes, promote adhesion to the substrate, and can provide flame-retardant properties.

According to a further or alternative embodiment, the UVC indicator comprises a solvent-based photochromic ink, which solvent-based photochromic ink comprises a phosphate, wherein the phosphate is selected from triphenyl phosphate or tripolyphosphate. Triphenyl phosphate or tripolyphosphate is added to ink compositions to optimize the rheological properties (flowability, viscosity), adhesion, stability, and color distribution.

Triphenyl phosphate is preferably used in ink as a softener or plasticizer. Due to its chemical structure, triphenyl phosphate can reduce the intermolecular forces of attraction between, for example, the pigment particles, thereby decreasing the viscosity of the ink and making it easier to process and apply. Additionally, triphenyl phosphate serves as an anti-foaming agent and can reduce the surface tension of the ink, enabling better wetting and spreading on the substrate. This is especially beneficial when printing on non-porous substrates such as plastics (e.g., polyethylene, polypropylene) where good wetting is essential for optimal adhesion.

Preferably, tripolyphosphate is used in ink as a complexing agent and stabilizer. It can interact with metal ions in the ink, such as calcium and magnesium, and complex them to prevent precipitation and changes in ink consistency. Moreover, tripolyphosphate can promote the dispersion of pigment particles in the ink by reducing their agglomeration. This results in a uniform dispersion of pigments in the ink, which improves the final color consistency and print quality.

According to a further or alternative embodiment, the UVC indicator comprises a solvent-based photochromic ink, wherein the solvent-based photochromic ink is a mixture of a solvent, a phosphate, and a UVC-sensitive pigment. The mentioned composition of a solvent-based photochromic ink with a mixture of a solvent, a phosphate, and a UVC-sensitive pigment is considered ideal due to the complementary benefits and functionalities of each component. The solvent acts as the medium in which the other components are dissolved. It provides the right viscosity and flowability of the ink, allowing it to be easily applied and spread. Moreover, it facilitates the drying of the ink on the substrate. The phosphate in the ink can play various roles, including viscosity control, pH stabilization, stabilization of pigments and dyes, improved adhesion, and reactive properties. The phosphate contributes to the overall stability and functionality of the ink. The UVC-sensitive pigment is the key to the photochromic properties of the ink. Under influence of UVC radiation, this pigment undergoes a color change, which fulfills the essential function of the UVC indicator. This contributes to the observation and measurement of UVC radiation, which is important for various applications. By combining these three components, a synergistic effect is achieved where the solvent acts as the medium that efficiently brings the other components together and aids in the application, while the phosphate and the UVC-sensitive pigment add the desired chemical and optical properties to the ink. This makes the proposed composition ideal as an ink for an effective UVC indicator.

According to a preferred embodiment, the UVC indicator is transparent before exposure to UVC radiation. The transparency of the UVC indicator allows users to quickly and easily see whether a surface or object has been exposed to UVC radiation for disinfection. If it is transparent, this means that no disinfection has taken place yet. The transparent state of the UVC indicator thus prevents confusion or doubt about the disinfection status. Users can immediately see whether the intended disinfection procedure has been carried out and whether the surface is safe. Moreover, the presence of the transparent indicator in the proximity of or above the object will minimally disrupt the sterilization process. Although the indicator will absorb a portion of the UVC radiation, the effect on the disinfection process is minimal. The object will still be effectively disinfected. It ensures an effective and efficient sterilization, while simultaneously enabling users to confidently verify whether the sterilization process has been properly executed. Preferably, the UVC indicator will change from transparent to blue after exposure to UVC radiation.

According to a preferred embodiment, the UVC indicator has a thickness of at least 1 µm and at most 10 mm, preferably at least 2 µm and at most 9 mm, more preferably at least 3 µm and at most 8 mm, even more preferably at least 4 µm and at most 7 mm, even more preferably at least 5 µm and at most 6 mm, even more preferably at least 6 µm and at most 5 mm, even more preferably at least 7 µm and at most 4 mm, even more preferably at least 8 µm and at most 3 mm, even more preferably at least 9 µm and at most 2 mm, and even more preferably at least 10 µm and at most 1 mm.

According to a preferred embodiment, the UVC indicator will display the level of exposure to UVC radiation. Displaying the level of exposure to UVC radiation by the UVC indicator is preferably achieved through a color change or other visual signal based on the exposure to UVC radiation. This mechanism is designed to provide information about the dose of UVC radiation to which the packaging has been exposed. The color change or visual indication is directly linked to the amount of UVC radiation absorbed. As exposure to UVC radiation increases, for example, the color of the indicator changes, allowing users to infer the extent to which disinfection has occurred. This is of great importance because it provides a direct visual indication of the effectiveness of the disinfection process. Users can quickly and easily assess based on the color change whether a particular surface or object has been adequately exposed to the UVC radiation and whether disinfection has been successfully carried out. This increases the reliability and effectiveness of the disinfection process, contributing to a safer and more hygienic environment. It also facilitates compliance with disinfection protocols and guidelines, which is crucial for ensuring health and safety in various applications.

To this end, the UVC indicator can be designed to change color as exposure to UVC radiation increases. For example, from an initial color (e.g., blue) or colorless (transparent) to another color (e.g., purple or pink) after a certain level of exposure.

Alternatively, the indicator can display subtler color shades that change based on the level of exposure. This can be achieved by using colors that gradually become more intense as exposure to UVC radiation increases. Using different types of ink with unique reactions to UVC radiation is an option in this regard. This ink can change color, fluoresce, or display other visual signals when exposed to UVC radiation. The UVC indicator can be divided into stripes, fields or areas that show different responses to exposure to UVC radiation. Each part can have a different color scheme or visual pattern depending on the level of exposure.

According to an embodiment, the UVC indicator is designed to be versatile and can take various forms, including designs, texts, images, lines, or even an entire surface. This flexible design makes it possible to adapt the indicator to specific needs and applications. Whether it's about integrating the indicator into existing packaging, displaying specific messages, creating visual patterns with lines, or covering an entire surface, the UVC indicator retains its functionality to effectively display the level of exposure to UVC radiation.

According to a preferred embodiment, the UVC indicator is a single-passage indicator. The single-passage indicator is suitable for changing color once after exposure to UVC radiation. A single-passage indicator has the ability to respond very accurately to exposure to UVC radiation. This one-time color change serves as a reliable indicator of whether the object is disinfected or not. The single discoloration after exposure to UVC radiation provides a robust and permanent signal that does not fade or change during transport. It provides a lasting indication of disinfection, regardless of the conditions during transport. Users can easily distinguish whether the disinfection process has occurred by simply looking at the change in color, thereby eliminating potential for confusion.

According to an alternative embodiment, the UVC indicator is a multi-passage indicator. The multi-passage indicator is suitable for changing color after exposure to UVC radiation and returning to its original state after a certain period in the absence of UVC radiation, preferably after a maximum of 10 minutes. The indicator's ability to return to its original state after a certain period in the absence of UVC radiation, allows for the reuse of the indicator. This allows the same indicator to be used multiple times, resulting in a more sustainable and cost-effective usage. Users can benefit from repeated use of the same indicator, reducing the need for frequent replacements. The ability to reuse the indicator minimizes the amount of waste that would otherwise be generated by the constant need to replace indicators. This contributes to an environmentally friendly approach and promotes sustainability.

According to a preferred embodiment, the bag comprises a second compartment, where the second compartment is equipped with the UVC indicator. This design has clear advantages because the UVC indicator is separated from the first compartment, which contains the object to be sterilized. This prevents the indicator from disrupting the disinfection process of the object. This separation ensures that the UVC indicator changes color when exposed to UVC radiation and remains visible at all times, which is crucial for accurate monitoring of the disinfection. The UVC radiation will disinfect the object in the first compartment without hindrance. At the same time, the indicator remains well-positioned to accurately register and visually display the disinfection. In this way, the functionality of the indicator is maximized without hindering the disinfection procedure. Additionally, this ensures that the UVC indicator receives the same dose of radiation as the object in the first compartment, since part of the radiation is absorbed by the walls of the bag. By placing the UVC indicator in a second compartment, as is the case with the object in the first compartment, the radiation will first pass through the wall of the bag before it reaches the UVC indicator. In this scenario, both the object and the UVC indicator will receive an equal amount of radiation, enhancing the accuracy of the UVC indicator's indication. In a further embodiment, the UVC indicator is adhered to the inner wall of the second compartment of the bag, and preferably the second compartment also provides space for other objects or documents (e.g., for identification, chain of custody, traceability, etc.) that do not necessarily need to be disinfected. Here, maintaining disinfection in this compartment plays a less crucial role or is not applicable at all.

According to a further embodiment, a label is provided with the UVC indicator, where the label is provided in the second compartment. Hereafter, the UVC indicator, which is provided on the label, is also called a UVC indicator label. This increases the durability and reliability of the indicator. Because the UVC indicator label is placed in the second compartment, the chance of interference with the object to be disinfected is minimized. This allows the UVC radiation to reach the first compartment unhindered and carry out the disinfection process effectively. The second compartment acts as a protective environment for the UVC indicator label, shielding it from potential external influences that could affect the functioning of the indicator. Preferably, the second compartment can be made completely sealable, minimizing the risk of loss or misplacement of the label. The UVC indicator provided on the label remains clearly visible in the second compartment, allowing users to easily monitor the disinfection status at all times. This contributes to a transparent disinfection process and increases confidence in its effectiveness. Placing the UVC indicator label in the second compartment finally makes the implementation straightforward and user-friendly. The label can be easily applied, removed, and replaced for optimal functionality.

According to a further embodiment, the first compartment and the second compartment are separated from each other, preferably the first compartment and second compartment are hermetically separated from each other. This separation can be achieved by means of a partition, a zipper between the compartments, a hook-and-loop fastener, or by melting seams. By using a partition, the object to be disinfected is completely shielded from the second compartment, in which the UVC indicator is placed. This ensures that the disinfection process remains isolated and focused on the object, without any external interference. The hermetic separation minimizes the chance of possible contamination or cross-contamination. This contributes to the hygiene and reliability of the disinfection process. The hermetic separation of the compartments ensures that the UVC indicator remains protected and retains its functionality, without influence from external factors. By the hermetic separation, the UVC indicator retains its integrity and remains clearly visible. This provides a clear and reliable indication of the disinfection status of the object.

In an alternative or further preferred embodiment, the bag includes (besides a first and a second) a third compartment for documents such as, among others, identification, chain of custody, traceability, etc. that do not necessarily have to be disinfected or sterilized. Here, the first, second, and third compartments are hermetically separated from each other so that no shadow formation can occur in the first and second compartment and no non-sterile or infected material or air can enter the second and first compartment after disinfection and/or sterilization. The third compartment ensures that the object and the documents mentioned, among other things, remain together and/or can be combined at all times, which prevents objects from being mixed up during transport, and guarantees that the right objects are used for the right applications. In this context, the second compartment includes only a UVC indicator, whereby the UVC indicator can be stuck against the inner wall of the second compartment, or whereby the UVC indicator can be provided loose in the second compartment. However, to avoid that the object to be sterilized or the UVC indicator would be shielded from the UVC radiation, and thus either an incomplete sterilization would occur of the object (which would lead to a non-sterile object of which there is no knowledge, which may be reused or contaminate other objects) and/or at the indicator (which would lead to a seemingly non-sterile object that needs to be re-irradiated). Through the mutual separation, these situations are avoided.

According to a preferred embodiment, the bag includes an appendage separate from the first compartment, wherein the appendage is provided with the UVC indicator. Because the UVC indicator is placed in a separate appendage, this appendage can be made from different types of materials because this part does not need to be disinfected. This increases flexibility in material choice and makes it possible to select the most suitable material on which the indicator will be provided. Because the appendage is separated from the first compartment, it does not obstruct the passage of UVC radiation to the object to be disinfected. This ensures that the effectiveness of the disinfection process remains unaffected. Placing the UVC indicator in a separate appendage facilitates its implementation and integration. This makes adjustments and reconfigurations easier and ensures a smooth user experience. The UVC indicator in the appendage remains clearly visible, allowing users to easily monitor the disinfection status. This ensures a transparent disinfection process and increases confidence in its effectiveness.

According to a preferred embodiment, the bag includes an appendage separate from the first compartment, wherein the appendage is provided with the UVC indicator, wherein the UVC indicator is provided on a sticker, which sticker is provided on the appendage of the bag.

According to an embodiment, the bag includes an appendage separate from the first compartment, wherein the appendage is provided with the UVC indicator, wherein the UVC indicator is printed on the appendage.

According to an embodiment, the packaging comprises a bag and a UVC indicator.

According to an embodiment, the packaging comprises a bag and a UVC indicator, which bag comprises at least two opposing walls, the at least two walls defining a first compartment for receiving the sterilizable object.

According to an embodiment, the packaging comprises a bag and a UVC indicator, which UVC indicator comprises a photochromic ink suitable for changing color after exposure to UVC radiation. In this way, it is indicated whether or not the object is disinfected or sterilized.

According to an embodiment, the packaging comprises a bag and a UVC indicator. The bag comprises at least two opposing walls, the at least two walls defining a first compartment for receiving the sterilizable object. The UVC indicator comprises a photochromic ink suitable for changing color after exposure to UVC radiation.

According to a preferred embodiment, the packaging comprises a bag, which bag comprises at least two opposing walls, wherein each wall has a thickness of at least 10 µm and at most 60 µm and is made of a UVC transmissive thermoplastic polymer, the at least two walls defining a first compartment for receiving the sterilizable object; and a UVC indicator, which UVC indicator comprises a photochromic ink suitable for changing color after exposure to UVC radiation. The inventors have surprisingly found that this proposed packaging is extremely suitable for the sterilization or disinfection of objects and to transport them safely thereafter without the risk of recontamination. This will help prevent the spread of infections. First of all, the use of UVC transmissive thermoplastic polymer allows efficient disinfection and sterilization due to its permeability to UVC radiation. This feature is crucial to ensure that objects are thoroughly cleaned and rid of germs and bacteria. In addition, the ideal balance between flexibility and durability is achieved by precisely specifying the wall thickness of at least 10 µm and at most 60 µm. This allows the packaging to retain its strength while the UVC radiation effectively penetrates and does its job. The presence of this indicator, using photochromic ink that changes color after exposure to UVC radiation, provides a clear visual representation of the disinfection process. This contributes to the confidence in the effectiveness of the disinfection procedure, allowing users to quickly and easily verify if the desired level of disinfection has been achieved. This cleverly designed packaging combines effective disinfection with a reliable indication, making it an optimal packaging for the safe and efficient disinfection and sterilization of objects. The emphasis here is on both health and hygiene aspects.

According to a preferred embodiment, the packaging comprises a bag, which bag comprises at least two opposing walls, wherein each wall has a thickness of at least 10 µm and at most 60 µm and is made of a UVC transmissive thermoplastic polymer, the at least two walls defining a first compartment for receiving the sterilizable object. The bag further comprises a second compartment, where the second compartment is equipped with the UVC indicator. The UVC indicator comprises a photochromic ink suitable for changing color after exposure to UVC radiation.

According to a preferred embodiment, the packaging comprises a bag, which bag comprises at least two opposing walls, wherein each wall has a thickness of at least 10 µm and at most 60 µm and is made of a UVC transmissive thermoplastic polymer, the at least two walls defining a first compartment for receiving the sterilizable object. The bag further comprises an appendage, separate from the first compartment, wherein the appendage is provided with the UVC indicator. The UVC indicator comprises a photochromic ink suitable for changing color after exposure to UVC radiation.

The inventors have surprisingly found that this proposed packaging is extremely suitable for the sterilization or disinfection of objects and to transport them safely thereafter without the risk of recontamination. This will help prevent the spread of infections. First of all, the use of UVC transmissive thermoplastic polymer allows efficient disinfection and sterilization due to its permeability to UVC radiation. This feature is crucial to ensure that objects are thoroughly cleaned and rid of germs and bacteria. In addition, the ideal balance between flexibility and durability is achieved by precisely specifying the wall thickness of at least 10 µm and at most 60 µm. This allows the packaging to retain its strength while the UVC radiation effectively penetrates and does its job. The presence of this indicator, using photochromic ink that changes color after exposure to UVC radiation, provides a clear visual representation of the disinfection process. This contributes to the confidence in the effectiveness of the disinfection procedure, allowing users to quickly and easily verify if the desired level of disinfection has been achieved. The design of the packaging with opposing walls defines a first compartment, in which the sterilizable object is safely placed. This creates an isolated environment in which the disinfection process can take place accurately without risk of recontamination.

Moreover, the packaging includes a bag, which bag is provided with a separate part to accommodate the indicator in or on. To this end, the packaging includes a second compartment or an appendage which will contain the UVC indicator. It creates a clear separation between the object to be disinfected and the UVC indicator or other objects or items, for example, certain documents that belong to the object but should not be disinfected. This minimizes the risk of cross-contamination and ensures a hygienic disinfection process. The separate location of the UVC indicator offers flexibility for different indicator types or adaptations, allowing the design to be adjusted to specific needs. This cleverly designed packaging combines effective disinfection with a reliable indication, making it an optimal packaging for the safe and efficient disinfection and sterilization of objects. The emphasis here is on both health and hygiene aspects. Finally, in this way, a UV indicator can be replaced after use (remove the old, discolored one, and add a new one), which allows for the reuse of the packaging.

According to an embodiment, the object to be disinfected will undergo a first disinfection step. After this first disinfection step, the object is placed in the packaging and then exposed to UVC radiation. There are various methods available for this initial disinfection step. For example, but not limited to, chemical disinfection or thermal disinfection. In chemical disinfection, disinfectants are used to kill or inactivate microorganisms on surfaces and materials. In thermal disinfection, heat is applied using steam, hot air, or autoclaves to kill microorganisms. This approach ensures that there is no chance of cross-contamination in the logistics chain. By applying the UVC radiation, additional assurance is provided that the object remains free from any pathogens or contaminants until the packaging is opened.

In a second aspect, the invention relates to a use according to the first aspect for transporting and disinfecting sterilizable objects, preferably for transporting and disinfecting sterilizable medical objects and food items.

This use of the proposed packaging is exceptionally advantageous due to the capacity to enable and maintain disinfection and sterilization of objects. Specifically for medical items and food, this use offers several important advantages.

During the transport of medical items, such as instruments or equipment, preventing recontamination is crucial to avoid the spread of infections. This is essential for patient safety and healthcare practices.

For food products, hygiene is of the utmost importance. The ability to safely disinfect food and keep it that way increases overall food safety and reduces the risk of food-related illnesses. This contributes to an improved quality of the food chain.

By maintaining an effective disinfection or sterilization throughout the entire duration of storage in the packaging, perishable medical or food products will stay fresh longer. This contributes to minimizing spoilage, which in turn reduces waste and improves the efficiency of distribution.

In what follows, the invention is described by way of non-limiting examples or figures illustrating the invention, and which are not intended to and should not be interpreted as limiting the scope of the invention.

### DESCRIPTION OF THE DRAWINGS

The numbered elements in the figures are:
1 = packaging
2 = bag
3 = UVC indicator
4 = first compartment of the bag
5 = second compartment of the bag
6 = label
7 = appendage
8 = sticker
9 = the disinfectable/sterilizable or disinfected/sterilized object

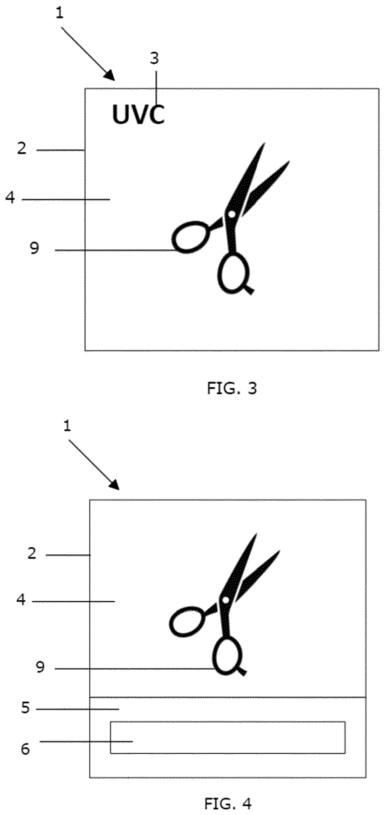

Figures 1, 2 and 3 show a schematic front view of different embodiments of the current invention. In this embodiment, the packaging 1 consists of a bag 2 and a UVC indicator 3. The bag will comprise at least two opposing walls, wherein each wall has a thickness of at least 10 µm and at most 60 µm and consists of a UVC transmissive thermoplastic polymer. The at least two walls define a first compartment 4 for receiving the sterilizable object 9. In these figures, it can be noted that the packaging 1 is exposed to UVC radiation, resulting in the discoloration of the UVC indicator 3. In figure 1, a colored rectangular UVC indicator 3 is shown. In figure 2, a colored circular UVC indicator 3 is visible. In figure 3, the letters "UVC" will appear as UVC indicator 3 after exposure to UVC radiation. In this embodiment, the UVC indicator 3 is printed or impressed on the bag 2, preferably on the outside of the first compartment 4 of the bag 2.

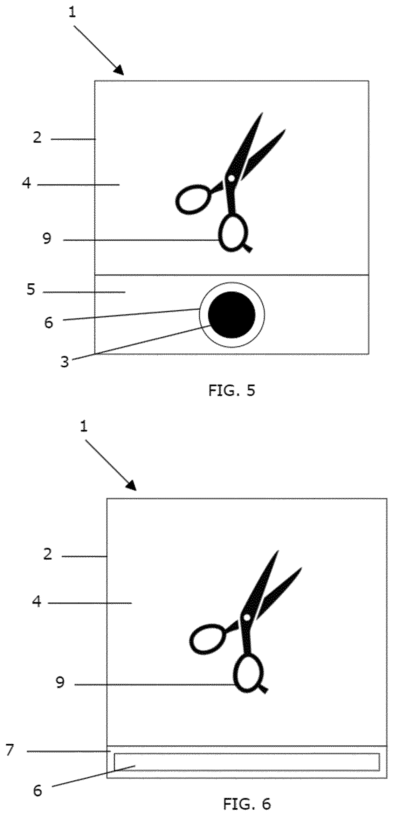

Figures 4 and 5 show a schematic front view of various embodiments of the present invention. In this embodiment, the packaging 1 consists of a bag 2 and a UVC indicator 3. The bag will comprise at least two opposing walls, wherein each wall has a thickness of at least 10 µm and at most 60 µm and consists of a UVC transmissive thermoplastic polymer. The at least two walls define a first compartment 3 for receiving the sterilizable object 9. Furthermore, the packaging 1 is provided with a second compartment 5. In this embodiment, the first compartment 4 and the second compartment 5 are separated from each other.

In Figure 4 the second compartment 5 is shown with the presence of a UVC indicator 3 on a rectangular label 6. This label 6 is located within the second compartment 5. In this specific configuration, the second compartment 5 is sealed, allowing the label 6 to lie loose in the compartment. It is important to note that the UVC indicator 3 itself is not directly observable, as it is displayed in a non-UVC irradiated form. The UVC indicator 3 is therefore transparent.

In figure 5, the UVC indicator 3 is located in the second compartment 5 and is placed on a circular label 6. In this embodiment, the second compartment is sealed, allowing the label 6 to move freely within the compartment. It is desirable that the UVC indicator 3 is a multi-passage indicator. Contrary to figure 4, in figure 5 the UVC indicator 3 is observable because, in this embodiment, it becomes visible after exposure to UVC radiation.

Figures 6, 7 and 8 show a schematic front view of different embodiments of the current invention. In this embodiment, the packaging 1 consists of a bag 2 and a UVC indicator 3. The bag will comprise at least two opposing walls, wherein each wall has a thickness of at least 10 µm and at most 60 µm and consists of a UVC transmissive thermoplastic polymer. The at least two walls define a first compartment 3 for receiving the sterilizable object 9. Further, the bag comprises an appendage 7, separate from the first compartment 4, wherein the appendage 7 is provided with the UVC indicator 3. The UVC indicator will also be able to take various forms in this embodiment.

In figure 6, a label 6, comprising the UVC indicator, is provided on the appendage 7. The UVC indicator 3 itself is not visible because in the embodiment it is displayed before exposure to UVC radiation, so the UVC indicator is transparent.

In figure 6, a label 6 is shown on which the UVC indicator 3 is attached, which is placed on the appendage 7. It is notable that the UVC indicator 3 is not directly observable by itself, as Figure 6 shows the situation before exposure to UVC radiation. As a result, the UVC indicator 3 is transparent.

Figure 7 shows several stickers 8 that have been applied to appendage 7. Each sticker 8 contains a UVC indicator 3. Preferably, each UVC indicator 3 will respond differently to a certain dose of UVC radiation, which will visually indicate the level of exposure to UVC radiation.

In figure 8, the colored UVC indicator 3 is visible, here in the form of the letters "UVC," which means that this packaging has been exposed to UVC radiation. Preferably, this UVC indicator 3 is printed or impressed on the appendage 7.

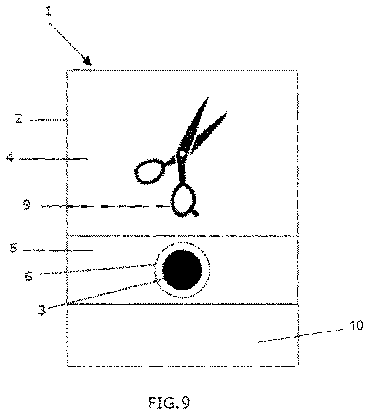

Figure 9 illustrates an embodiment in which the bag 1 comprises three hermetically separated compartments. A first compartment 2 suitable for retaining a sterilizable object 9. A second compartment 5 suitable for retaining a UVC indicator 3, which in this embodiment is provided on a round label 6. And a third compartment 10, for retaining documents that provide information about object 9, such as, for example, track-and-trace documents and/or information regarding the sterilization or disinfection level. In this embodiment, the sterilizable object 9 is separated from the UVC indicator 3 so that they cannot overlap, which would affect the irradiation of the sterilizable object 9 and/or the UVC indicator 3. It is also guaranteed that the UVC indicator 3 receives the same dose of radiation as the sterilizable object 9 because the UVC indicator, like the sterilizable object 9, is located in a compartment, At all times it is immediately clear which sterilizable object 9 is in the bag 2, which sterilization and/or disinfection requirements the sterilizable object 9 must comply with, and where the sterilizable object 9 needs to go, because this information is provided in the third compartment 10 of the bag 2.

## Claims

1. A packaging (1) for a sterilizable object (9), comprising:
a. a bag (2), which bag 2 comprises at least two opposing walls, wherein each wall has a thickness of at least 10 µm and at most 60 µm and is made of an ultraviolet-C (UVC) transmissive thermoplastic polymer, the at least two walls defining a first compartment (4) for receiving the sterilizable object (9); and
b. a UVC indicator (3), which UVC indicator (3) comprises a photochromic ink suitable for changing color after exposure to UVC radiation.

2. The packaging according to claim 1, wherein each wall of the bag (2) is made from a polypropylene, preferably from a biaxially oriented polypropylene, more preferably from a fluorinated ethylene propylene.

3. The packaging according to claim 1 or 2, wherein the UVC indicator (3) is a single-passage indicator, which single-passage indicator is suitable for changing color once upon exposure to UVC radiation.

4. The packaging according to any of the preceding claims 1 or 2, wherein the UVC indicator (3) is a multi-passage indicator, which multi-passage indicator is suitable for changing color after exposure to UVC radiation and returning to an original state after a certain period in the absence of UVC radiation, preferably after a maximum of 10 minutes.

5. The packaging according to any of the preceding claims 1 to 4, wherein the UVC indicator (3) is transparent before exposure to UVC radiation.

6. The packaging according to any of the preceding claims 1 to 5, wherein the UVC indicator (3) has a thickness of at least 1 µm and at most 10 mm.

7. The packaging according to any of the preceding claims 1 to 7, wherein the first compartment (4) is provided with at least one opening.

8. The packaging according to any of the preceding claims 1 to 7, wherein the bag (2) includes a second compartment (5), wherein the second compartment (5) is provided with the UVC indicator (3), which is located inside the bag.

9. The packaging according to claim 8, wherein a label 6 is provided with the UVC indicator (3), wherein the label (6) is provided in the second compartment (5).

10. The packaging according to claim 8 or 9, wherein the first compartment (4) and the second compartment (5) are separated from each other, preferably the first compartment (4) and second compartment (5) are hermetically separated from each other.

11. The packaging according to any of the preceding claims 1 to 7, wherein the bag (2) includes an appendage (7) separate from the first compartment (4), wherein the appendage (7) is provided with the UVC indicator (3).

12. The packaging according to claim 11, where the UVC indicator (3) is provided on a sticker (8), which sticker (8) is provided on the appendage (7) of the bag (2).

13. The packaging according to any of the preceding claims 1 to 12, **characterized in that** the UVC indicator (3) shows the level of exposure to UVC radiation.

14. The packaging according to any of the preceding claims 1 to 13, **characterized in that** the UVC indicator comprises a mixture containing between 15% by weight and 35% by weight of a UVC-sensitive pigment, and at least 65% by weight of a solvent.

15. The packaging according to any one of claims 1 to 14, **characterized in that** the bag (2) comprises a second compartment (5) and a third compartment (10), wherein the second compartment (5) is provided with the UVC indicator (3), which is located inside the bag, and wherein the third compartment (10) is suitable for receiving items associated with the object to be sterilized, preferably documentation regarding identification of the object, chain of custody for the object, track and trace for the object, and/or manual for the object.
